# EUROPEAN PATENT APPLICATION

(11) **EP 3 995 535 A1**
(43) Date of publication of application: **11.05.2022**
(21) Application number: 20834962.1
(22) Date of filing: 01.07.2020
(51) Int. Cl.: C08K 5/12, C08L 27/06, C08L 101/00, C07C 67/08, C07C 69/80

(54) **PHTHALIC ACID ESTER COMPOSITION**

(30) Priority: 03.07.2019 JP 2019124253
(71) Applicant: Mitsubishi Chemical Corporation, Chiyoda-ku Tokyo 100-8251 (JP)
(72) Inventor: KIYOOKA Kazuhiko, Tokyo 100-8251 (JP); MATSUZAKA Koki, Tokyo 100-8251 (JP); TANAKA Yoshiyuki, Tokyo 100-8251 (JP)
(74) Representative: Ter Meer Steinmeister & Partner
(86) International application number: PCT/JP2020/025907
(87) International publication number: WO 2021/002406

(57) **Abstract**

An object of the present invention is to provide a phthalic acid ester composition ensuring that when used as a plasticizer as an alternative to DOP, various performances are well-balanced and furthermore, that the hydrolysis resistance is excellent and even when used under the conditions causing hydrolysis, an unpleasant odor is hardly generated. The present invention relates to a phthalic acid ester composition containing (2-ethylheptyl)(2-propylhexyl)phthalate, bis(2-ethylheptyl)phthalate, and bis(2-propylhexyl)phthalate.

## Description

### TECHNICAL FIELD

The present invention relates to a phthalic acid ester composition. In addition, the present invention relates to a plasticizer, a resin composition and a molded body, each containing a phthalic acid ester composition, and further relates to a production method of a phthalic acid ester composition.

### BACKGROUND ART

Conventionally, an alcohol ester of phthalic acid is used as a plasticizer for a resin such as vinyl chloride-based resin (Patent Literature 1). Among others, dioctyl phthalate (DOP) which is a phthalic acid ester of 2-ethylhexanol which is an alcohol having a number of carbon atoms of 8 (hereinafter referred to as C8 alcohol) achieves a good balance of various performances such as flexibility, heat resistance, cold resistance, oil resistance and processability, etc. in the case of being used as a plasticizer and particularly, is widely used as a plasticizer for a vinyl chloride-based resin.

However, due to recent chemical regulation and self-regulation associated therewith, substitution of DOP with other plasticizers is progressing. The strongest requirement for the alternative plasticizer is that the above-described various performances are close to those of DOP.

For this reason, diisononyl phthalate (DINP) which is a phthalic acid ester of isononyl alcohol which is an alcohol having a carbon number of 9 (hereinafter referred to as C9 alcohol) is often used as the alternative plasticizer to DOP. Because, DINP is a phthalic acid ester similar to DOP and achieves the above various performances which are close to those of DOP.

Here, the isononyl alcohol serving as a raw material of DINP is usually a C9 alcohol mixture composed of various branched isomers. As the phthalic acid ester of isononyl alcohol, there are known two types, i.e., DINP under Cas. No. 28553-12-0 which is a reaction product of isononyl alcohol under Cas. No. 27458-94-2 with a phthalic acid, and DINP under Cas. No. 68515-48-0 that is a reaction product of isononyl alcohol under Cas. No. 68526-84-1 with a phthalic acid.

As for other alternative plasticizers, in view of supply convenience or cost, etc., bis(2-ethylhexyl)terephthalate (DOTP) which is a terephthalic acid ester of 2-ethylhexanol which is a C8 alcohol is used.

### CITATION LIST

### PATENT LITERATURE

Patent Literature 1: JP-A-H10-120515

### SUMMARY OF INVENTION

### TECHNICAL PROBLEM

However, DINP has a problem that when a synthetic resin composition, such as vinyl chloride-based resin composition, containing this ester is used for applications involving contact with an alkali component-containing substance such as concrete, hydrolysis occurs to cause an unpleasant odor. In addition, isononyl alcohol as a raw material alcohol thereof is produced in small quantities, compared with 2-ethyl-hexanol as a raw material alcohol of DOP, and there are concerns about supply. Also, when switching from DOP to DINP further progresses, supply of DINP may become tight.

Furthermore, in the case of using DOTP as a plasticizer, since part of performances such as light resistance or oil resistance, etc. are inferior to DOP, its use as an alternative to DOP is sometimes difficult in some applications of a soft vinyl chloride product.

An object of the present invention is to provide a phthalic acid ester composition ensuring that when used as a plasticizer as an alternative to DOP, various performances such as flexibility, heat resistance, oil resistance and processability, etc. are well-balanced and furthermore, that the hydrolysis resistance is excellent and even when used under the conditions causing hydrolysis, for example, involving contact with a substance containing an alkali component, an unpleasant odor is hardly generated.

### SOLUTION TO PROBLEM

As a result of intensive studies to attain the object above, the present inventors have found that when a phthalic acid ester composition containing (2-ethylheptyl)(2-propylhexyl)phthalate, bis(2-ethylheptyl)phthalate, and bis(2-propylhexyl)phthalate is used as a plasticizer, the above-described problems can be solved. The present invention has been accomplished based on this finding.

Namely, the gist of the present invention is described below.
[1] A phthalic acid ester composition containing (2-ethylheptyl)(2-propylhexyl)phthalate, bis(2-ethylheptyl)phthalate, and bis(2-propylhexyl)phthalate.
[2] The phthalic acid ester composition according to [1], further containing at least one selected from the group consisting of diisononyl phthalate (DINP), dioctyl phthalate (DOP), diisodecyl phthalate (DIDP), and di-(2-propylheptyl)phthalate (DPHP).
[3] The phthalic acid ester composition according to [1] or [2], containing a total of 10 wt% or more of (2-ethylheptyl)(2-propylhexyl)phthalate, bis(2-ethylheptyl)phthalate, and bis(2-propylhexyl)phthalate.
[4] A plasticizer containing the phthalic acid ester composition according to any one of [1] to [3].
[5] A resin composition containing the phthalic acid ester composition according to any one of [1] to [3] and a resin.
[6] The resin composition according to [5], wherein the resin is a vinyl chloride-based resin.
[7] A molded body obtained by molding the resin composition according to [5] or [6].
[8] A method for producing the phthalic acid ester composition according to [1], containing performing an esterification reaction of a mixture of 2-ethylheptyl alcohol and 2-propylhexyl alcohol with at least one selected from the group consisting of phthalic acid and phthalic anhydride.
[9] The production method of a phthalic acid ester composition according to [8], wherein the mixture is produced by a cross-aldol reaction and a hydrogenation reaction of butyraldehyde and valeraldehyde.

### EFFECTS OF INVENTION

The phthalic acid ester composition of the present invention ensures that when used as a plasticizer, various performances such as flexibility, heat resistance, oil resistance and processability, etc. are well-balanced and furthermore, that the hydrolysis resistance is excellent and even when used under the conditions causing hydrolysis, for example, involving contact with a substance containing an alkali component, an unpleasant odor is hardly generated. Therefore, the phthalic acid ester composition of the present invention can be suitably used as a plasticizer for a vinyl chloride-based resin, etc., as an alternative to DOP.

### DESCRIPTION OF EMBODIMENTS

The embodiments of the present invention are described in detail below.

1. Phthalic Acid Ester Composition and Production Method Thereof

The phthalic acid ester composition of the present invention is a phthalic acid ester composition containing (2-ethylheptyl)(2-propylhexyl)phthalate, bis(2-ethylheptyl)phthalate, and bis(2-propylhexyl)phthalate.

The phthalic acid ester composition of the present invention can be produced by an esterification reaction of a mixture of 2-ethylheptyl alcohol and 2-propylhexyl alcohol with at least one selected from the group consisting of phthalic acid and phthalic anhydride.

In the phthalic acid ester composition of the present invention, the content of each phthalate may be arbitrarily determined according to the required plasticizer performances, but the contents of respective phthalates are preferably set to be from 5 to 45 wt% for bis(2-ethylheptyl)phthalate, from 5 to 45 wt% for bis(2-propylhexyl)phthalate, and from 10 to 90 wt% for (2-ethylheptyl)(2-propylhexyl)phthalate. In particular, the contents of respective phthalates are preferably set to be from 20 to 30 wt% for bis(2-ethylheptyl)phthalate, from 20 to 30 wt% for bis(2-propylhexyl)phthalate, and from 40 to 60 wt% for (2-ethylheptyl)(2-propylhexyl)phthalate, because the composition ensures no unpleasant odor generation and excellent hydrolysis resistance, which are characteristics of the present invention, and has plasticizer performances close to those of DOP which is a general-purpose plasticizer.

The phthalic acid ester composition having such a constitution is obtained by an esterification reaction of a mixture of 2-ethylheptyl alcohol and 2-propylhexyl alcohol having a mixing ratio of 1:5 to 5:1 (by weight) with at least one selected from the group consisting of phthalic acid and phthalic anhydride.

Also, (alcohol mixture above)/(at least one selected from the group consisting of phthalic acid and phthalic anhydride) in the esterification reaction is, in molar ratio, usually from 2 to 3, preferably from 2.05 to 2.95, and more preferably from 2.1 to 2.9.

In this connection, in the present description, the "at least one selected from the group consisting of phthalic acid and phthalic anhydride" is sometimes referred to as "phthalic acid and/or phthalic anhydride".

The esterification reaction is not particularly limited in its reaction conditions, etc. as long as a desired ester product can be produced. For example, a mixture of 2-ethylheptyl alcohol and 2-propylhexyl alcohol can be reacted with phthalic acid and/or phthalic anhydride in the presence of an esterification catalyst, preferably in the presence of an acid catalyst such as sulfuric acid, or in the presence of a metallic catalyst, etc. such as organic titanium compound or organic tin compound, by heating the system at a temperature not more than the boiling point of an ester product in a nitrogen atmosphere as needed with removing water produced by the esterification reaction.

The reaction temperature of the esterification reaction varies depending on the catalyst, etc. used but, in view of the stability of the ester product and the dehydration efficiency, etc., may be usually from 100 to 250°C, and preferably from 120 to 230°C. After the completion of reaction, purification is performed by removing unreacted carboxylic acids, unreacted alcohols and catalysts, etc. remaining in the system by use of a purification method such as vacuum distillation (stripping), steam distillation, alkali neutralization, water washing and filtration, etc., whereby a target phthalic acid ester composition can be obtained. At the time of purification, quality-improving assistants such as decolorant, deodorant, adsorbent, filter aid, etc. may be used.

Whether a target composition is obtained can be confirmed by various analysis methods. For example, confirmation by gas chromatography (GC) or gas chromatography-mass spectroscopy analysis (GC-MS), etc. is simple and preferred.

Here, the mixture of 2-ethylheptyl alcohol and 2-propylhexyl alcohol used in the present invention may be a mixture obtained by mixing separately-produced 2-ethylheptyl alcohol and 2-propylhexyl alcohol, but is more preferably a mixture obtained by a cross-aldol reaction and a hydrogenation reaction of a mixed aldehyde of butyraldehyde and valeraldehyde, because the number of production steps is small.

The mixing ratio of butyraldehyde and valeraldehyde in the mixed aldehyde is preferably from 1:4 to 4:1 (by weight), and more preferably from 2:3 to 3:2 (by weight).

The mixed aldehyde may be a mixed aldehyde obtained by mixing butyraldehyde produced by hydroformylation reaction of propylene with valeraldehyde produced by hydroformylation reaction of butene or may be a mixed aldehyde produced by hydroformylation reaction of a mixture of propylene and butene.

The catalyst in the hydroformylation reaction includes, for example, rhodium complex catalysts containing, as a ligand, an organic phosphine such as trialkylphosphine, triarylphosphine and (mono- or di-)alkyl(di- or mono-)arylphosphine; a monophosphate such as trialkyl phosphite, triaryl phosphite and (mono- or di-)alkyl(di- or mono-)aryl phosphite; a cyclic or acyclic organic phosphite such as bisphosphite and polyphosphite of the above; etc. In addition, a given amount of an organic phosphate may be allowed to be present together with such a complex catalyst.

The hydroformylation reaction can be performed in a liquid phase using a solvent according to an ordinary method and is usually performed at a temperature of 15 to 200°C under a pressure of 0.1 to 300 kg/cm² with a molar ratio (H₂/CO) of hydrogen and carbon monoxide of 10/1 to 1/10 for a reaction time of 0.01 to 20 hours.

In addition, the amount used of the rhodium complex catalyst may be from 1 to 100,000 ppm in terms of rhodium atom. The amount used of the organic phosphorus compounds serving as the ligand may be from 1 to 10,000 times by mol in terms of the atomic ratio of phosphorus to rhodium. As for the preparation of the rhodium complex catalyst, a complex may be formed in a reaction system by separately supplying a rhodium source compound and an organic phosphorus compound to the inside of a reaction system, or after forming a complex previously from a rhodium source compound and an organic phosphorus compound outside of a reaction system, the complex may be added to the reaction system.

The cross-aldol reaction of the mixed aldehyde can also be performed in a liquid phase or a gas phase according to an ordinary method and, for example, in the case of a liquid phase, the reaction is usually performed in an alkali aqueous solution such as caustic soda at a temperature of 60 to 120°C under not less than a saturation pressure of the liquid at that temperature, for example, under a pressure ranging from normal pressure to 10 kg/cm².

Furthermore, the hydrogenation reaction of the mixed aldehyde obtained by the cross-aldol reaction can also be performed in a liquid phase or a gas phase according to an ordinary method and, usually, is performed in the presence of a catalyst, for example, a catalyst containing a Group VIII metal such as nickel, palladium and platinum, etc., a solid catalyst containing a reduction mixture of copper oxide and zinc oxide, a copper-chromium-based catalyst, or a copper-chromium-manganese-barium-based catalyst, usually at a temperature of 50 to 300°C under a hydrogen pressure ranging from normal pressure to 200 kg/cm². The reaction product obtained by the hydrogenation reaction is subjected to separation and purification, for example, by distillation, etc.

The phthalic acid ester composition of the present invention preferably contains a total of 10 wt% or more, more preferably from 50 to 100 wt%, still more preferably from 70 to 100 wt%, and particularly preferably from 80 to 100 wt%, of (2-ethylheptyl)(2-propylhexyl)phthalate, bis(2-ethylheptyl)phthalate, and bis(2-propylhexyl)phthalate.

The phthalic acid ester composition of the present invention may further contain other phthalic acid esters. Other phthalic acid esters include diisononyl phthalate (DINP), dioctyl phthalate (DOP), diisodecyl phthalate (DIDP), and di-(2-propylheptyl)phthalate (DPHP), etc., and the phthalic acid ester composition of the present invention may contain at least one of these esters.

The phthalic acid ester composition of the present invention can be used as a plasticizer.

### 2. Plasticizer

The plasticizer of the present invention contains the phthalic acid ester composition of the present invention. The plasticizer of the present invention preferably contains a total of 10 wt% or more, more preferably from 50 to 100 wt%, still more preferably from 70 to 100 wt%, and particularly preferably from 80 to 100 wt%, of (2-ethylheptyl)(2-propylhexyl)phthalate, bis(2-ethylheptyl)phthalate, and bis(2-propylhexyl)phthalate.

The plasticizer of the present invention may contain, in addition to the phthalic acid ester composition of the present invention, at least one plasticizer selected from the group consisting of an adipic acid ester-based plasticizer, a phosphoric acid ester-based plasticizer, a trimellitic acid ester-based plasticizer, a benzoic acid ester-based plasticizer, and a polyester-based plasticizer.

### 3. Resin Composition and Molded Body Thereof

The resin composition of the present invention contains the phthalic acid ester composition of the present invention and a resin. The resin (a material to be plasticized) used in the resin composition of the present invention is not particularly limited and includes resins and rubbers conventionally using a plasticizer, for example, a vinyl chloride-based resin, a styrene-based resin, a cellulose-based resin, an acrylic resin, a vinyl-based resin such as chlorinated polyethylene, rubbers such as chloroprene rubber, styrene-butadiene rubber, acrylonitrile-butadiene rubber and urethane rubber, and various thermoplastic elastomers, etc. Among these, a vinyl chloride-based resin is particularly preferred.

The vinyl chloride-based resin includes, for example, a vinyl chloride homopolymer, a copolymer of vinyl chloride and ethylene, propylene, styrene, vinyl acetate, vinylidene chloride, maleic acid or an ester thereof, a copolymer of vinyl chloride and acrylonitrile, acrylic acid or an ester thereof, and a copolymer of vinyl chloride and methacrylic acid or an ester thereof.

In the resin composition of the present invention, (2-ethylheptyl)(2-propylhexyl)phthalate, bis(2-ethylheptyl)phthalate, and bis(2-propylhexyl)phthalate are preferably contained in a proportion of, in total, from 0.1 to 300 parts by weight, more preferably in a proportion of 1 to 200 parts by weight, and still more preferably in a proportion of 5 to 100 parts by weight, per 100 parts by weight of the resin.

When the total content of respective phthalates is not less than the range above, sufficient plasticity can be imparted to the resin composition. On the other hand, when the total content of respective phthalates is not more than the range above, reduction of various physical properties such as mechanical strength or bleed-out may not occur.

In this connection, as long as the object of the present invention is not impaired, the resin composition of the present invention may contain at least one plasticizer selected from the group consisting of an adipic acid ester-based plasticizer, a phosphoric acid ester-based plasticizer, a trimellitic acid ester-based plasticizer, a benzoic acid ester-based plasticizer, and a polyester-based plasticizer. Also, the resin composition of the present invention may contain additives usually used for a vinyl chloride-based resin, for example, a stabilizer, an antioxidant, an ultraviolet absorber, a lubricant, an antistatic agent, an antifogging agent, a mold release agent, a flame retardant, a coloring agent, a filler, etc.

The resin composition of the present invention may be mold-processed into a molded body. In this case, the methods for molding can be roughly classified into two methods. One is a method where a vinyl chloride-based resin obtained by suspension polymerization is mixed with the plasticizer of the present invention and other compounding agents, melt-kneaded in a kneader such as mill roll, calender roll, Banbury mixer, Brabender and extruder, etc., and then molded in a desired shape by extrusion molding, injection molding, hollow molding, compression molding, etc. to form a molded body. The other one is a method where a vinyl chloride-based resin obtained by emulsion polymerization or fine suspension polymerization is mixed with the plasticizer of the present invention and other compounding agents to make a viscous plastisol, molded in a desired shape by dip molding, slush molding, rotational molding, etc., and then gelled at a high temperature to form a molded body.

### EXAMPLES

The present invention is described more specifically below by referring to Experimental Examples, Examples and Comparative Examples.

### [Odor Measurement]

### (Odor of Isononyl Alcohol)

Odors of isononyl alcohols (Cas. No. 27458-94-2 and Cas. No. 68526-84-1) were measured at 20°C using a 5-standard odor set for panel selection manufactured by Daiichi Yakuhin Sangyo Co., Ltd. by adopting, as subjects, 5 persons judged as having no problems with the odor perception. At the odor measurement, evaluation was performed in accordance with the criteria shown in Table 1. All subjects perceived an unpleasant odor.

Next, 30 ml of water, 170 ml of ethanol and 100 mg of potassium hydroxide as a base were added to a solution obtained by dissolving 0.4 g of diisononyl phthalate (DINP) in 20 ml of acetone, made into a uniform solution and then left standing at 25°C for 1 day. With respect to this solution, the same 5 persons smelled the odor at ordinary temperature in the same manner, as a result, perceived an unpleasant odor similar to the odor of isononyl alcohol.

From this result, it is determined that the odor perceived when using DINP for applications involving contact with a substance containing an alkali component is derived from isononyl alcohol serving as a raw material alcohol resulting from hydrolysis of DINP with an alkali component. Also, the cause of this unpleasant odor of isononyl alcohol is presumed to be attributable to the fact that it is a mixture of isomers having various kinds of branching degrees.

### (Odor of Raw Material Alcohol)

Odors of respective raw material alcohols (a C9 alcohol mixture, isononyl alcohol Cas. No. 27458-94-2, isononyl alcohol Cas. No. 68526-84-1) were smelled at 20°C using a 5-standard odor set for panel selection manufactured by Daiichi Yakuhin Sangyo Co., Ltd. by adopting, as subjects, 10 persons judged as having no problems with the odor perception, and scored in accordance with the criteria of Table 1, and the total score was taken as the evaluation score. The results are shown in Table 2. In this connection, the C9 alcohol mixture was prepared according to the following method.

### <Production Method of C9 Alcohol Mixture>

A mixed aldehyde composed of 56.5 wt% of n-butyraldehyde and 43.5 wt% of n-valeraldehyde was put in a round bottom flask having an inner volume of 3 L which had been set to a nitrogen atmosphere in advance and heated up to a temperature of 80°C, and 957 g of an aqueous 2 wt% caustic soda solution was added dropwise over 1 hour. A condensation reaction was allowed to proceed at a temperature of 80°C for 3.5 hours, and the reaction product was then cooled and phase-separated to obtain a condensation reaction product.

Subsequently, 20 g of a cylindrical nickel-chromium-based hydrogenation catalyst having a diameter of 5 mm and a length of 5 mm (previously reduced in a hydrogen stream at 150°C) and 500 g of the condensation reaction product were put in a SUS-made high-pressure reaction vessel having an inner volume of 1 L filled under a nitrogen atmosphere, and a liquid-phase hydrogenation reaction was allowed to proceed at a temperature of 150°C under a hydrogen pressure of 5.0 MPa to produce a mixed alcohol.

The mixed alcohol was subjected to reduced-pressure distillation under the conditions of a reflux ratio of 10/1, 130°C and 1.4 kPa by using a 20-tray Oldershaw distillation apparatus to obtain a C9 alcohol mixture containing 49.0 wt% of 2-ethylheptyl alcohol and 49.1 wt% of 2-propylhexyl alcohol. Note that each component was confirmed by GC measurement.

**[Table 1]**

| | Intensity of Odor | Offensiveness of Odor |
|---|---|---|
| 5 | very strong odor | very strong discomfort |
| 4 | strong odor | strong discomfort |
| 3 | medium odor | medium discomfort |
| 2 | weak odor | mild discomfort |
| 1 | substantially odor free | substantially discomfort free |

**[Table 2]**

| Evaluator | C9 Alcohol Mixture | | Isononyl Alcohol Cas. No. 27458-94-2 | | Isononyl Alcohol Cas. No. 68526-84-1 | |
|---|---|---|---|---|---|---|
| | Intensity of Odor | Offensiveness of Odor | Intensity of Odor | Offensiveness of Odor | Intensity of Odor | Offensiveness of Odor |
| A | 2 | 2 | 4 | 3 | 3 | 4 |
| B | 3 | 2 | 3 | 3 | 3 | 4 |
| C | 3 | 2 | 3 | 3 | 3 | 2 |
| D | 2 | 2 | 3 | 3 | 3 | 3 |
| E | 3 | 3 | 4 | 4 | 4 | 4 |
| F | 2 | 2 | 3 | 3 | 3 | 2 |
| G | 3 | 3 | 4 | 4 | 4 | 4 |
| H | 2 | 1 | 4 | 3 | 3 | 3 |
| I | 2 | 1 | 3 | 3 | 3 | 4 |
| J | 2 | 2 | 4 | 4 | 4 | 4 |
| Average | 2.4 | 2 | 3.5 | 3.3 | 3.3 | 3.4 |

It is seen from Table 1 and Table 2 that with respect to the odor of a raw material alcohol (a C9 alcohol mixture) generated, for example, due to letting a resin product using the phthalic acid ester composition of the present invention as a plasticizer be used under the conditions causing hydrolysis, both the intensity of odor and the offensiveness of odor were rated the lowest by all evaluators, compared with the odor of a raw material alcohol (isononyl alcohol) generated, for example, due to letting a resin product using DINP as a plasticizer be used under the conditions causing hydrolysis.

This reveals that when the phthalic acid ester composition of the present invention was used as a plasticizer and even when used under the conditions causing hydrolysis, an unpleasant odor was hardly generated.

### [Example 1]

### (Production of Phthalic Acid Ester Composition)

Into a flask equipped with a stirrer and a cooling tube-attached oil/water separation device, 66 g (0.45 mol) of phthalic anhydride, 163 g (1.1 mol) of the C9 alcohol mixture, and 0.09 mL of tetraisopropyl titanate were charged and reacted by raising the temperature up to 220°C with removing the reaction product water in the course of temperature rise and reducing the pressure inside the system as needed to maintain the reflux state. When the acid value of the reaction solution in the system reached 0.17 mgKOH/g, the heating was stopped, and the excess alcohol content was removed with increasing the degree of pressure reduction up to 1.3 kPa.

Subsequently, the reaction solution was cooled to 90°C and kept at that temperature, and an aqueous 0.3% calcium hydroxide solution having mixed therein 0.19 g of diatomaceous earth was added thereto, followed by stirring for 20 minutes. Thereafter, the pressure in the system was gradually reduced to 0.5 kPa at 140°C and after removing water and alcohol over 5 hours, the residue was filtered to obtain a phthalic acid ester composition containing 23.4 wt% of bis(2-ethylheptyl)phthalate, 24.6 wt% of bis(2-propylhexyl)phthalate, and 48.4 wt% of (2-ethylheptyl)(2-propylhexyl)phthalate.

Each component was confirmed by GC and GC-MS measurements under the following measurement conditions. The acid value of the obtained phthalic acid ester composition as measured in conformity with JIS K6751 was 0.003 mgKOH/g.

### (GC)

Apparatus: Shimadzu GC-17A
Column: GL Science Inc., InertCap-1, non-polar, 0.32 mm×60 m×0.25 µm
Temperature: 80°C (0 min, 10°C/min) → 300°C (15 min)
Ti=300°C, Td=300°C
Gas: N₂=27 cm/sec
Injection amount: 0.2 µL
Split: 1:33
Quantification Method: area normalization method

### (GC-MS)

Apparatus: Shimadzu GC/MS-QP2010Ultra
Column: Supelco Inc., Supelcowax-10, strongly polar, 0.32 mm×60 m×0.25 µm
Temperature: 40°C (4 min, 10°C/min) → 210°C (49 min)
Ti=230°C, ion source=250°C, IF=250°C
Gas: He=40 cm/sec
Injection amount: 0.2 µL
Split: 1:10
Pretreatment: none
Measurement mode: CI

### <Hydrolysis Resistance>

In a 500-ml glass bottle, 25 ml of distilled water and 170 ml of acetone were put and mixed to obtain uniform Solution 1. To Solution 1, 20 ml of a solution prepared by dissolving 3.8 g of the obtained phthalic acid ester composition in 20 ml of acetone was added to obtain Solution 2. After adding 20 ml of 2 N hydrochloric acid to Solution 2, the resulting solution was divided into two portions to obtain Solution 3.

Next, as a blank test, one portion of Solution 3 was titrated with a 0.1 N KOH/methanol solution. The other portion of Solution 3 was heated at 50°C for 6 hours using an aluminum block heater and then titrated with a 0.1 N KOH/methanol solution, and the hydrolysis rate of the obtained phthalic acid ester composition was calculated based on the difference from the blank. The results are shown in Table 3.

### [Example 2]

Except for using a mixture of DINP [Cas. No. 28553-12-0 (using isononyl alcohol of Cas. No. 27458-94-2 as a raw material alcohol)]/phthalic acid ester composition obtained in Example 1 = 1/1 (by weight) in place of the phthalic acid ester composition obtained in Example 1, the hydrolysis rate of the mixture was calculated in the same manner as in Example 1, and the results are shown in Table 3.

### [Example 3]

Except for using a mixture of DINP above/phthalic acid ester composition obtained in Example 1 = 9/1 (by weight) in place of the phthalic acid ester composition obtained in Example 1, the hydrolysis rate of the mixture was calculated in the same manner as in Example 1, and the results are shown in Table 3.

### [Comparative Example 1]

The hydrolysis rate of DINP was calculated in the same manner as in Example 1 except that DINP above was used in place of the phthalic acid ester composition obtained in Example 1, and the results are shown in Table 3.

**[Table 3]**

| | | Example 1 | Example 2 | Example 3 | Comparative Example 1 |
|---|---|---|---|---|---|
| Phthalic acid ester-based plasticizer | Phthalic acid ester composition of the present invention (wt%) | 100 | 50 | 10 | 0 |
| | DINP (wt%) | 0 | 50 | 90 | 100 |
| Hydrolysis rate (%) | | 0.8 | 1.6 | 2.2 | 2.7 |

### [Example 4]

### (Preparation of Vinyl Chloride-Based Resin Composition)

A vinyl chloride-based resin composition composed of 100 parts by weight of a vinyl chloride resin (average polymerization degree: 1300), 50 parts by weight of, as a plasticizer, the phthalic acid ester composition obtained in Example 1, 3 parts by weight of epoxidized soybean oil, 3 parts by weight of a CaZn-based stabilizer, and 20 parts by weight of calcium carbonate was obtained. The obtained vinyl chloride-based resin composition was evaluated for processability by the following method. The results are shown in Table 4.

### <Processability Test of Vinyl Chloride-Based Resin Composition>

With respect to the obtained vinyl chloride-based resin composition, the time until reaching a maximum torque at a temperature of 160°C and a rotor rotational speed of 50 rpm was measured using LABO PLASTMILL. A shorter time indicates better processability.

In addition, the vinyl chloride resin composition obtained above was premixed, then mixed for 5 minutes by means of a twin roll mill controlled to a temperature of 155°C, preheated at a temperature of 180°C under a pressure of 1.96 MPa for 2 minutes, and subsequently, held for 1 minute under a pressure of 15 MPa to prepare a press sheet having a thickness of 1 mm. The press sheet obtained was evaluated by the following evaluation methods. The results are shown in Table 4.

### <Flexibility>

The tensile strength (MPa) of the press sheet obtained was measured in conformity with JIS K6723.

### <Volatility (Heat Resistance)>

The press sheet obtained was heated under the post-heating tensile test conditions in conformity with JIS K6723. The weight decrease amount of the press sheet after heating was measured based on the press sheet before heating. The measured value was taken as the weight decrease amount (volatilization amount) of the plasticizer, and the rate of decrease (%) from the original weight of the plasticizer was determined.

### <Oil Resistance>

The press sheet obtained was immersed in oil under the oil resistance test conditions in conformity with JIS K6723. The weight decrease amount of the press sheet after immersion was measured based on the press sheet before immersion. The measured value was taken as the weight decrease amount (elution amount into oil) of the plasticizer, and the rate of decrease (%) (elution rate of plasticizer into oil) from the original weight of the plasticizer was determined.

### [Comparative Example 2]

The evaluations were performed in the same manner as in Example 4 except that DINP (Cas. No. 28553-12-0 [using isononyl alcohol of Cas. No. 27458-94-2 as a raw material alcohol)] was used as the plasticizer, and the results are shown in Table 4.

### [Comparative Example 3]

The evaluations were performed in the same manner as in Example 4 except that DOP (Cas. No. 117-81-7) was used as the plasticizer, and the results are shown in Table 4.

**[Table 4]**

| Measurement Item | Unit | Example 4 | Comparative Example 2 (DINP) | Comparative Example 3 (DOP) | Remarks |
|---|---|---|---|---|---|
| Processability | sec | 4.3 | 4.3 | 4.3 | Shorter time indicates better processability. |
| Flexibility | MPa | 11.0 | 10.9 | 10.5 | Smaller tensile strength indicates higher flexibility. |
| Volatility (heat resistance) | % | 2.2 | 1.2 | 5.1 | Smaller rate of decrease indicates lower volatility. |
| Oil resistance | % | 0.6 | 0.9 | 1.0 | Smaller rate of decrease indicates better oil resistance |

It is seen from Tables 3 and 4 that compared with DINP conventionally used as an alternative to DOP, in the phthalic acid ester composition of the present invention, the hydrolysis resistance is excellent and the flexibility, volatility, oil resistance and processability, which are general plasticizer performances, are substantially equal and balanced. This reveals that similarly with DINP, the phthalic acid ester composition of the present invention is sufficiently usable as an alternative to DOP.

While the present invention has been described in detail and with reference to specific embodiments thereof, it will be apparent to one skilled in the art that various changes and modifications can be made therein without departing from the spirit and scope of the invention. This application is based on Japanese Patent Application (Patent Application No. 2019-124253) filed on July 3, 2019, the contents of which are incorporated herein by way of reference.

## Claims

1. A phthalic acid ester composition comprising (2-ethylheptyl)(2-propylhexyl)phthalate, bis(2-ethylheptyl)phthalate, and bis(2-propylhexyl)phthalate.

2. The phthalic acid ester composition according to claim 1, further comprising at least one selected from the group consisting of diisononyl phthalate (DINP), dioctyl phthalate (DOP), diisodecyl phthalate (DIDP), and di-(2-propylheptyl)phthalate (DPHP).

3. The phthalic acid ester composition according to claim 1 or 2, comprising a total of 10 wt% or more of (2-ethylheptyl)(2-propylhexyl)phthalate, bis(2-ethylheptyl)phthalate, and bis(2-propylhexyl)phthalate.

4. A plasticizer comprising the phthalic acid ester composition according to any one of claims 1 to 3.

5. A resin composition comprising the phthalic acid ester composition according to any one of claims 1 to 3 and a resin.

6. The resin composition according to claim 5, wherein the resin is a vinyl chloride-based resin.

7. A molded body obtained by molding the resin composition according to claim 5 or 6.

8. A method for producing the phthalic acid ester composition according to claim 1, comprising performing an esterification reaction of a mixture of 2-ethylheptyl alcohol and 2-propylhexyl alcohol with at least one selected from the group consisting of phthalic acid and phthalic anhydride.

9. The production method of a phthalic acid ester composition according to claim 8, wherein the mixture is produced by a cross-aldol reaction and a hydrogenation reaction of butyraldehyde and valeraldehyde.
